# EUROPEAN PATENT SPECIFICATION

(11) **EP 1 521 841 B1**
(45) Date of publication and mention of the grant of the patent: **14.04.2010**
(21) Application number: 03739241.2
(22) Date of filing: 20.06.2003
(51) Int. Cl.: C12Q 1/00, C12Q 1/68, C12Q 1/70, G01N 33/53, G01N 33/566, G01N 33/567

(54) **METHOD FOR IDENTIFYING INDIVIDUAL ACTIVE ENTITIES FROM COMPLEX MIXTURES**
VERFAHREN ZUR IDENTIFIZIERUNG EINZELNER AKTIVER EINHEITEN AUS KOMPLEXEN GEMISCHEN
PROCEDE PERMETTANT D'IDENTIFIER DES ENTITES ACTIVES INDIVIDUELLES DANS DES MELANGES COMPLEXES

(30) Priority: 11.07.2002 US 395038 P
(43) Date of publication of application: 13.04.2005
(73) Proprietor: The American National Red Cross, Rockville, MD 20855 (US)
(72) Inventor: HAMMOND, David, J., Laytonsville, MD 20882 (US); LATHROP, Julia, Tait, Falls Church, VA 22046 (US); SARKAR, Jolly, North Potomac, MD 20878 (US); GHEORGHIU, Liliana, Cambridge, MA 02139 (US)
(74) Representative: Bates, Philip Ian
(86) International application number: PCT/US2003/019584
(87) International publication number: WO 2004/007757

(56) References cited:
- EP-A- 0 639 584
- WO-A-01/40265
- WO-A-03/089922
- WO-A1-92/00091
- WO-A2-01/40265
- US-A- 5 482 867
- US-A- 5 482 867
- JONES C M ET AL: "Synthetic macrophage activating peptides derived from the N-terminus of human MCF" BIOCHEMICAL AND BIOPHYSICAL RESEARCH COMMUNICATIONS, ACADEMIC PRESS INC. ORLANDO, FL, US, vol. 199, no. 1, 28 February 1994 (1994-02-28), pages 20-25, XP002353091 ISSN: 0006-291X
- KELLAR KATHRYN L; KALWAR RIZWAN R; DUBOIS KIMBERLY A; CROUSE DENNIS; CHAFIN WILLIAM D; KANE BETH-ELLEN: "Multiplexed fluorescent bead-based immunoassays for quantitation of human cytokines in serum and culture supernatants" CYTOMETRY, vol. 45, no. 1, 2001, pages 27-36, XP009066240 USA

## Description

### FIELD OF THE INVENTION

This invention pertains to methods of screening a mixture for active entities.

### BACKGROUND OF THE INVENTION

Conventional proteomics seeks to generate a comprehensive identity profile of the entire proteome of an organism and, through analysis of this information, to identify potential diagnostic and therapeutic entities. Currently, the dominant technologies for resolving protein mixtures are two-dimensional gel electrophoresis and multi-dimensional liquid chromatography, both coupled to mass spectrometry. An example of the power of this approach is the resolution and identification of 1,484 proteins in yeast (Washburn et al., Nat. Biotechnol., 9(3): 242-2471 (2001)). A quantitative methodology for protein separation and identification is isotope coded affinity tag (ICAT), developed by Aebersold and colleagues (Smolka et al., Anal. Biochem., 297(1): 25-312 (2001)). ICAT involves the site-specific, covalent labeling of proteins with isotopically normal or heavy reagents to quantitate levels of protein expression. Yet another example of methodology that separates and identifies proteins is a global version of the yeast two-hybrid screening assay developed by Uetz et al. (Uetz et al., Nature, 403(6770): 623-627 (2000)) and Ito et al. (Ito et al., Proc. Natl. Acad. Sci. USA, 98(8): 4569-4574 (2001)), which identified over 4,000 protein-protein interactions in *Saccharomyces cerevisiae.* Although these approaches for separating and identifying proteins are powerful, they do not identify the cellular functions of the fractionated proteins.

Complex protein mixtures also have been separated on libraries of combinatorially-generated ligands immobilized on beads. Combinatorial and synthetic chemistry techniques well-known in the art can generate libraries of millions of ligands, (Lam et al., Nature, 354: 82-84 (1991) and International (PCT) Patent Application WO 92/00091) each of which may have the capacity to bind to molecules. A library of linear hexamer ligands made with 18 of the natural amino acids, for example, contains 34 x 10⁶ different structures. When amino acid analogs and isomers are also included, the number of potential structures is practically limitless. Each bead essentially has millions of copies of a single structure on its surface and different beads contain different sequences. Moreover, the total number of beads in a library may be enormous.

Following exposure of an entity molecule to a combinatorial library, the entity will bind through affinity interactions to specific ligands within the library. At equilibrium, the concentration of the entity on the support, e.g., a bead bearing an affinity ligand, will be dependent on the affinity constant and the concentration of the ligand and the entity. In published screening methodologies, the detection of the bound entity and hence its ligand can be straightforward when a purified, radiolabeled initial entity is used (Mondorf et al., J. Peptide Research, 52: 526-536 (1998)). Other methods include detection by an antibody against the entity (Buettner et al., International Journal of Peptide & Protein Research, 47: 70-83 (1996); Furka et al., International Journal Peptide Protein Research, 37(6): 487-493 (1991); and Lam et al., (1991) *supra*). Ligands to multiple entities can be detected using beads immobilized on an adhesive in combination with a subtractive screening method. This is referred to as the QuASAR method (International (PCT) Patent Application WO 01/40265) and was used to detect ligands that bound to virus and prion protein.

In a related but distinct methodology using a bead-based library, the entity protein in its normal physiological environment is incubated with a peptide ligand library bound to beads. Following fractionation,the beads are immobilized and arrayed in a thin gel of low melting-point agarose to create a crude "array" of ligands. A protein-binding membrane (nitrocellulose or PVDF) is laid on the gel, and the proteins are eluted from the beads under a variety of conditions and captured in the same relative position as they were in the gel on the membrane by unidirectional capillary transfer of solvent diffusing through the gel (similar to a Southern transfer of DNA). As all of the proteins that were present in the original mixture that bound to ligands immobilized on beads are present on the membranes, the membranes themselves can be stripped and reprobed for different, known proteins.

In the foregoing methods, in which a library of combinatorially-generated ligands immobilized onto beads is used, entity proteins are simply separated and identified based on their binding to the ligands and not on their biochemical or biological function. Therefore, there exits a need in the art for methods that separates and identify proteins from complex mixtures based on their chemical, physical, biological, and/or biochemical function and not merely on their ability to bind a ligand within the library.

The invention provides such methods. This and other objects and advantages of the invention, as well as additional inventive features, will be apparent from the description of the invention provided herein.
WO92/0009 A1 discloses a library of bio-oligamers of defined size and known composition. US 5 482 867 A discloses methods and compositions for immobilising anti-ligands, such as antibodies or antigens, hormones or hormone receptors, oligonucleotides, and polysaccharides on surfaces of solid substrates.
EP 0639584 A discloses a method for the preparation of high density peptide (or other polymer) libraries, and for screening such libraries for molecules having the capacity to recognise targets of choice. WO03/089922 A, of the present applicant, filed before but published after the filing date of the present application, discloses a method for detecting ligands and targets in a mixture. WO01/40265 A2 discloses methods of identifying a ligand for a target molecule.

### BRIEF SUMMARY OF THE INVENTION

The invention provides methods of screening a mixture for active entities, which method comprises (i) providing a plurality of different ligands, wherein each ligand is attached to a support to form a plurality of ligand-support complexes, (ii) contacting the ligand-support complexes with a mixture comprising a plurality of entities under conditions that allow at least one entity to bind to at least one ligand-support complex, thereby forming more than one entity-ligand-support complex, (iii) separating more than one entity-ligand-support complex from the unbound entities; (iv) assaying the activity of the entity, wherein the entity may be dissociated partially or completely from an entity-ligand-support complex separated in step (iii), and wherein the activity assayed is not solely binding of the entity to the ligand-support complex; (v) detecting the activity, and (vi) selecting at least one entity-ligand-support-complex that bound the entity that exhibited the detected activity, whereupon a mixture is screened for active entities, wherein the step of separating more than one entity-ligand support complex from the unbound entities does not comprise transferring the entity from a first matrix to a second matrix.

The ligands may be selected from the group consisting of proteins, peptides, amino acids, nucleic acids, carbohydrates, lipids, drugs, synthetic inorganic compounds, synthetic organic compounds, isoforms of any of the foregoing, cells, bacteria, viruses, yeast, and combinations of any of the foregoing. If the ligands are peptides, the peptides may be generared by combinatorial approaches.

The support may comprise a material selected from the group consisting of polymethacrylates, polyacrylates, agarose, polyacrylamides, dextran, cellulose, polysaccharides, nitrocellulose, silicon, styrene, polyethylene-coated polystyrene, metal, polyvinyldifluoride, nylon, and combinations of any of the foregoing.

The mixture may be a biological fluid, an environmental extract, or a composition comprising chemical compounds. If the mixture is a biological fluid, it may be selected from the group consisting of blood, plasma, pooled plasma, intermediates from plasma fractionation, serum, a cell homogenate, a tissue homogenate, a conditioned medium, a fermentation broth, cerebrospinal fluid, urine, saliva, milk, ductal fluid, tears, perspiration, lymph, semen, umbilical cord fluid, and amniotic fluid. The biological fluid may be a plasma-derived fraction comprising antibodies and anti-idiotype antibodies. The biological fluid may be obtained from a host afflicted with a disease. If the mixture is an environmental extract, the environmental extract may be selected from the group consisting of a soil extract, an extract from a naturally-occurring body of water, a sample of ice, air, ash, rock, or permafrost, and a swab from a building. If the mixture is a composition comprising chemical compounds, the composition comprising chemical compounds may comprise natural or synthetic chemical compounds.

The entities may be selected from the group consisting of proteins, peptides, drugs, antibodies, cells, synthetic molecules, organic compounds, protein complexes, bacteria, viruses, and fungi.

The activity may be a biological, physical, chemical, biochemical activity or any combination thereof. The activity may be an enzyme activity or an inhibition of an enzyme activity, or may be an effect on a cell, a cell population, a tissue, or a whole organism. If the activity is the effect on a cell or a cell population, the effect may be selected from the group consisting of cell migration, cell proliferation, cell death, cell differentiation, cell cycle entry, cell cycle arrest, apoptosis, cell lysis, growth arrest, cell survival, a change in an intracellular signaling pathway, antigen expression, gene upregulation, gene downregulation, and a phenotypic change in response to an agent. If the activity is the effect on a cell, a cell population, tissue, or whole organism, the cell, cell population, tissue, or whole organism may be diseased; the cell, cell population, tissue or whole organism may be diseased with cancer, diabetes, an autoimmune disease, osteoporosis, or lung disease, infected with a parasite, virus, or bacteria, wounded, burned, scarred, or in a state of healing.

Methods of the present invention may further comprise, after step (iii) and before step (iv), a sub-pooling step, wherein at least one separated entity-ligand-support complex and the ligand-support complexes are separated into several pools. In addition, the method may further comprise, after the sup-pooling step and before step (iv), an eluting step, wherein the entities of the multiple entity-ligand-support complexes are dissociated from the complexes, and the ligand-support complexes are subsequently removed from the pools; it may also further comprise, after the sub-pooling step and before step (iv), a step, wherein a semi-solid or viscous material is added to each pool, wherein the entity of at least one entitity-ligand-support complexes dissociates from the complex and diffuses into the material, thereby forming a concentration gradient of the entity, wherein the concentration of the entity gradually decreases as the distance from the ligand-support complex from which the entity dissociated increases.

Methods of the present invention may further comprises a step (vii) of determining the chemical identity of at least one ligand to which at least one entity exhibiting the detected activity binds. In addition, the method may further comprise the steps (viii) providing multiple copies of at least one ligand identified in step (vii), (ix) attaching each copy to a support, thereby obtaining multiple ligand-support complexes, (x) contacting the ligand-support complexes with a composition comprising multiple copies of at least one entity exhibiting the detected activity under conditions that allow the ligand-support complexes to bind multiple copies of at least one entity exhibiting the detected activity, thereby forming multiple entity-ligand-support complexes, and (xi) dissociating the entities from the entity-ligand-support complexes, thereby recovering the entities from the composition. In such a method, the composition of step (x) may be the same as the mixture of step (ii). Methods of the present invention may further comprise (xii) determining the chemical or physical identity of the entity exhibiting the detected activity.

Methods of the present invention may further comprise characterising the active entity and its binding ligand.

### DETAILED DESCRIPTION OF THE INVENTION

The invention provides methods of screening a mixture for active entities, which method comprises (i) providing a plurality of different ligands, wherein each ligand is attached to a support to form a plurality of ligand-support complexes, (ii) contacting the ligand-support complexes with a mixture comprising a plurality of entities under conditions that allow at least one entity to bind to at least one ligand-support complex, thereby forming more than one entity-ligand-support complex, (iii) separating more than one entity-ligand-support complex from the unbound entities; (iv) assaying the activity of the entity, wherein the entity may be dissociated partially or completely from an entity-ligand-support complex separated in step (iii), and wherein the activity assayed is not solely binding of the entity to the ligand-support complex; (v) detecting the activity, and (vi) selecting at least one entity-ligand-support-complex that bound the entity that exhibited the detected activity, whereupon a mixture is screened for active entities, wherein the step of separating more than one entity-ligand support complex from the unbound entities does not comprise transferring the entity from a first matrix to a second matrix.

The term "mixture," as used herein refers to any collection comprising more than one entity, wherein the term "entity" as used herein refers to any biological, chemical, or biochemical entity or target, such as a compound, molecule, virus, or cell. Preferably, the mixture is a collection of different entities; each having a different chemical identity, e.g., molecular formula, chemical structure, nucleotide sequence or amino acid sequence, or each having a different physical identity, e.g., spectral signal or conformation. More preferably, the mixture is a collection comprising a plurality of different entities. One of ordinary skill in the art appreciates that the mixture can comprise one or more copies of each entity having a different chemical or physical identity.

The entities comprising the mixture can be isolated from nature or synthetically produced, and can be organic or inorganic in nature (e.g., a synthetic inorganic compound or a synthetic organic compound). For example, the entity can be a drug or drug candidate (such as a small molecule drug candidate), a fertilizer component, an insecticide component, or a derivative, analogue, or enantiomer thereof. In addition, the entity can be endogenous or exogenous to any prokaryote or eukaryote, e.g., a bacterium, a fungus, yeast, a plant, or a mammal. Suitable entities for the inventive method include, but are not limited to, cells (e.g., stem cells or cells in culture), proteins, peptides, drugs, antibodies, synthetic molecules, organic compounds, protein complexes (e.g., blood clotting Factor XIII and fibrinogen or blood clotting Factor VIII and Von Willebrand Factor), bacteria, viruses, fungi, yeast, prions, amino acids, nucleic acids, carbohydrates, lipids, isoforms of any of the foregoing, and combinations of any of the foregoing. Preferably, the entities are proteins. Suitable protein entities include, for example, receptors, antibodies, immunogens, enzymes (e.g., proteases), and enzyme substrates. More preferably, the proteins are plasma-derived proteins. Most preferably, the plasma-derived proteins are immunoglobulins, e.g., IgG, IgM, IgA, IgE. The immunoglobulins can be from an organism in a diseased state, (and optionally not found in the plasma of a healthy subject) or produced as a result of the administration of an agent, e.g., a drug. Alternatively, it is preferred that the entities are cells. More preferably, the cells are stem cells.

The entity of the inventive methods can be obtained from any source, i.e., the mixture comprising the entities can be any complex mixture, such as extracts of soil, air, water, food, swabs for evaluating environmental contamination, intermediate or end-stage chemical reaction mixtures, and the like. The mixture comprising the entity can be a chemical or synthetic mixture and can be present in a combinatorial library and/or present in organic solvents under extreme conditions of pressure, temperature, etc. Preferably, the mixture is a biological fluid, an environmental extract, or a composition comprising chemical compounds.

By "biological fluid" is meant any aqueous solution that is derived from a prokaryotic or eukaryotic organism. The biological fluid can be obtained directly from the prokaryotic or eukaryotic organism, such as blood, lymph, tears, saliva, perspiration, and urine. Alternatively, the biological fluid can be obtained by culturing cells of the organism, such as fermentation broth and cell culture medium. Suitable biological fluids for use in the inventive method include, but are not limited to, blood, plasma, pooled plasma, intermediates of plasma fractionation, serum, a cell homogenate, a tissue homogenate, a conditioned medium, a fermentation broth, cerebrospinal fluid, urine, saliva, milk, ductal fluid, tears, perspiration, lymph, semen, umbilical cord fluid, and amniotic fluid. Preferably, the biological fluid is a plasma-derived fraction comprising antibodies and anti-idiotype antibodies. By "anti-idiotype" as used herein refers to an antibody that has an epitope that is specific for the antigen-determining region of another antibody. Also preferred is that the biological fluid is obtained from a host afflicted with a disease. The term "host" as used herein refers to any eukaryotic or prokaryotic organism, e.g., bacteria, virus, yeast, fungi, bird, reptile, and mammal. The disease, which afflicts the host, can be any disease including any condition, malady, infection, and the like. For instance, the disease can be cancer, diabetes, an autoimmune disease, osteoporosis, wound healing, liver regeneration, or lung disease. Alternatively, the disease can be an infection with a parasite, virus, or bacteria.

The term "environmental extract" as used herein refers to any sample taken from an environment. The environment can be a natural environment, such as a naturally-occurring body of water. Alternatively, the environment can be a man-made environment, such as a building. In this respect, the environmental extract can be a soil, soil extract, an extract from a naturally-occurring body of water, a sample of ice, air, ash, rock, or permafrost, or a swab from a building. The naturally-occurring body of water can be, for example, an ocean, a lake, a sea, a river, a swamp, a pond, a delta, or a bay. The environmental extract can alternatively be an extract from a water treatment center. The building can be any man-made building. Preferably, the building is contaminated with one or more toxic agents, such as sarin, soman, nerve poisons, explosive chemicals, pesticides, pathogens, VX, and blister agents.

The composition comprising chemical compounds can comprise natural or synthetic chemical compounds. For instance, the composition can be a chemical or synthetic mixture of reaction products. Alternatively, the composition can be present in a combinatorial library and/or present in organic solvents under extreme conditions of pressure, temperature, etc.

The term "ligand" as used herein refers to any biological, chemical, or biochemical agent, such as a compound, molecule, or cell that binds to an entity. The ligand can be isolated from natural or synthetically produced materials. The ligand can be endogenous or exogenous to a prokaryote or eukaryote, e.g. bacteria, a fungus, yeast, plant, or a mammal. Suitable ligands for the inventive methods include, but are not limited to, cells, bacteria, viruses, yeast, proteins, peptides, amino acids, nucleic acids, carbohydrates, lipids, drugs, synthetic inorganic compounds, synthetic organic compounds, antibody preparations (e.g., antibody fragments, chemically-modified antibodies, and the like), sugars, isoforms of any of the foregoing, and combinations of any of the foregoing.

Organic molecules include, for example, synthetic organic compounds typically employed as pharmacotherapeutic agents. Such molecules are, optionally, mass-produced by combinatorial synthetic methods or, more specifically, by strategic syntheses devised to arrive at specific molecules. Likewise, organic molecules also include natural products and analogues, whether extracted from their natural environment or strategically synthesized. The term "organic" as used herein is not intended to be limited to molecules comprised only of carbon and hydrogen, but rather is used in its broader sense as encompassing macromolecules of biological origin.

Preferably, the ligands are peptides. The term "peptide" as used herein refers to an entity comprising at least one peptide bond, and can comprise either D and/or L amino acids. Ideally, the ligand is a peptide consisting essentially of about 2 to about 10 amino acids (e.g., about 2, 3, 4, 5, 6, 7, 8, 9, or 10 amino acids). Desirably, the peptide ligands are generated by combinatorial approaches, i.e., techniques commonly employed in the generation of a combinatorial library, e.g., the split, couple, recombine method or other approaches known in the art (see, e.g., Furka et al., Int. J. Peptide Protein Res., 37: 487-493 (1991); Lam et al., Nature, 354: 82-84 (1991); International Patent Application WO 92/00091; and U.S. Patents 5,010,175, 5,133,866, and 5,498, 538). The expression of peptide libraries also is described in Devlin et al., Science, 249: 404-406 (1990). In peptide libraries, the number of discrete peptides of differing sequence increases dramatically with the number of coupling reactions performed, the size of the peptide, and the number of distinct amino acids utilized. For example, the random incorporation of 19 amino acids into pentapeptides produces up to 2,476,099 (19⁵) individual peptides of differing sequence (Lam et al., *supra*). Combinatorial methods allow generation of libraries of ligands directly on a support. Typically, the ligands are synthesized on particles of support media such that multiple copies of a single ligand are synthesized on each particle (e.g., bead), although this is not required in the context of the invention.

In the inventive methods, each of the ligands is attached to a support, thereby achieving formation of ligand-support complexes. The term "support" as used herein refers to any support matrix, such as those solid supports known in the art, which serve to immobilize the ligand. Suitable supports include, but are not limited to, membranes, filters, meshes, or particles comprising cellulose, acrylics, polyacrylamide or polyhydroxylated methacrylate polymers, polystyrene, dextran, agarose, polysaccharides, hydrophilic vinyl polymers, polymerized derivatives of any of the foregoing, and combinations of any of the foregoing, as well as any porous or non-porous matrix to which ligands can be directly attached or on which ligands can be synthesized. Preferably, the support is inert such that chemical reaction with the entity and/or the immobilized ligand is minimized. In this regard, the support desirably comprises a polymethacrylate, polyacrylate, agarose, a polyacrylamide, dextran, cellulose, a polysaccharide, nitrocellulose, silicon, styrene, metal, polyethylene-coated polystyrene, polyvinyldifluoride, nylon, or a combination of any of the foregoing. Particularly preferred support materials are polyethylene coated polystyrene and polymethacrylate. Various resins are commercially available, and, preferably, the support is a resin bead, such as a chromatographic resin bead.

Many solid supports displaying potential ligands are commercially available. Alternatively, the ligands of the inventive method can be indirectly attached or directly immobilized on the support using standard methods (see, for example, Harlow and Lane, Antibodies, Cold Spring Harbor Laboratory, Cold Spring Harbor, NY (1988); Biancala et al., Letters in Peptide Science 7(291): 297(2000); MacBeath et al., Science 289: 1760-1763 (2000); Cass et al., ed., Proceedings of the Thirteenth American Peptide Symposium; Leiden, Escom, 975-979 (1994); U.S. Patent 5,576,220; Cook et al., Tetrahedron Letters 35: 6777-6780 (1994); and Fodor et al., Science 251(4995): 767-773 (1991)). In one embodiment, the ligand(s) are synthesized on the surface of the support, which is advantageous in generating peptide libraries. The ligand(s) can be chemically conjugated to the support or can be attached via linkers, beta alanine, glycine, polymers containing glycine-serine, short chain hydrocarbons of the formula -(CH₂)-, polyethylene glycol, epsilon amino caproic acid, and linkers comprising -O(CH₂)n, wherein n is 1-30. If desired, the ligand(s) can be attached by one or by several different cleavable linkers, e.g., photolabile or acid labile moieties, enabling the selective detachment of a population of ligands for analysis. Ligands can be used, for example, as affinity purification media for proteins and enantiomeric separation (e.g., to concentrate, isolate, detect, characterize, quantify, or identify entities in a sample), as diagnostic therapeutic tools, catalysts and enhancers of chemical reactions, and as selective stabilizers of proteins.

Once formed, the ligand-support complexes are contacted with a mixture comprising a plurality of entities under conditions that allow at least one entity to bind to at least one ligand-support complex, thereby forming more than one entity-ligand-support complex. Such conditions, as recognized by one of ordinary skill in the art, depend upon the mixture (entities) and the ligands themselves, in addition to other factors, such as pH, temperature, contact time, salt concentration, and the like. Determination of suitable conditions that allow at least one entity to bind to at least one ligand-support complex is within the ordinary skill. In the present invention multiple entity-ligand. support complexes are formed.

After more than one entity-ligand support complex is formed, more than one entity-ligand-support complex and the ligand-support complexes are separated from the unbound entities. The term "unbound entities" as used herein refers to the entities of the mixture that do not bind or are loosely bound to any of the ligand-support complexes. Means of separating at least one entity-ligand-support complex from the unbound entities are known in the art and, include, for instance, centrifugation, serial dilution, filtration, dialysis; and washing in a chromatographic format.

The inventive methods can optionally comprise a sub-pooling step, wherein at least one entity-ligand-support complex and the ligand-support complexes are separated into several pools or sub-populations. Preferably, the sub-pooling step achieves on average about 10-500 entity-ligand-support complexes and ligand-support complexes per pool. More preferably, the average number of complexes per pool is 20-100. Most desirably, the complexes are sub-pooled, such that 50 complexes are present in each pool.

Furthermore, the inventive methods can optionally comprise, after the sub-pooling step, an eluting step, wherein the entities of the multiple entity-ligand-support complexes are dissociated from the complexes, and the ligand-support complexes are subsequently removed from the pools.

One of the advantages of this inventive method is that the entity being tested for activity dissociates from the ligand-support complex and is, thus, in an unbound state. It is therefore, uninhibited by the ligand and/or support and is free to interact with and act on the enzyme substrate or cells used in the activity assay. One of ordinary skill in the art recognizes that the degree of dissociation of the entity from the ligand-support complex need not be 100% or a complete dissociation, as it is possible that an activity of the entity can be detected with only some of the entity dissociated from the ligand-support complex.

Alternatively, the method could optionally further comprise, after the sub-pooling step, a step, wherein a semi-solid or viscous material is added to each pool, wherein the entity of at least one entity-ligand-support complex dissociates from the complex and diffuses into the material, thereby forming a concentration gradient of the entity, wherein the concentration of the entity gradually decreases as the distance from the ligand-support complex from which the entity dissociates increases. Suitable semi-solid materials for use in this method include, for instance, agarose, gelatin, glycerol, polyethylene glycol, acrylamide, and fibrin sealant. Preferably, the semi-solid material is 0.5% w/v agarose.

The advantages offered by this method also include the entity being in a free, unbound state, uninhibited by the ligand-support complex to interact with or act on an enzyme substrate or cells used in the activity assay. In addition, this method offers the advantage of being able to test the activity of the entity in a dose-dependent manner.

In the inventive methods, the mixture is screened for active entities by assaying at least one entity-ligand-support complex for an activity. The term "active entity" as used herein refers to any entity of the mixture that exhibits the assayed activity or a serendipitous result. In this regard, most entities of a given mixture have the potential of being an active entity, depending upon the particular activity being assayed in the method. For purposes herein, the active entity will also be active in the sense that the entity binds to a ligand-support complex. The assayed activity can be any biological, physical, chemical, or biochemical activity, provided that the activity results in a detectable signal, such as the enzymatic modification of a substrate. A chemical activity, e.g., an activity directly related to the chemical composition of the entity, can be employed. In other words, the entity can be identified by the presence of specific chemical subunits or moieties or chemical structures. Physical properties useful in detection methods include, for example, spectral signal, which can be determined via fluorescence or mass-spectrometry, respectively. The means of detection need not detect the activity of the entity alone, but can selectively identify activity or activities of more than one entity or of an entity complex, e.g., an entity complexed with other biological entities such as co-factors or enzymes.

Preferably, the activity can be an enzyme activity or inhibition of an enzyme activity. For example, the inventive method can comprise performing an enzyme activity assay to characterize an entity on the basis of biological activity. An enzyme substrate is applied to at least one entity-ligand-support complex under conditions which allow for enzymatic modification of the substrate by the entity to form a product The product is then detected, thereby identifying the presence of the entity. Alternatively, the lack of product formation could be detected in order to identify active entities that inhibit a given enzyme activity. Enzyme activity assays are further described in, for example, Haugland, *supra.*

The activity alternatively can be an effect on a cell, a cell population, a tissue, or a whole organism. In any of these instances, a cell-based assay is performed wherein at least one entity-ligand-support complex is contacted with cells on which the entity exerts some observable biologic effect. Purely by way of illustration, the preferred entity can be an antibacterial agent. The ligand of the inventive methods binds potential active sites of the antibacterial agent, thereby separating the antibacterial entity from a mixture (e.g., a library of potential therapeutics). A lawn of bacteria is applied to the entity, and the antibacterial entity is detected by zones of bactericidal activity. When the activity is an effect on a cell or a cell population, the effect can be, for instance, cell migration, cell proliferation, cell death, cell differentiation, cell cycle entry, cell cycle arrest, apoptosis, cell lysis, growth arrest, cell survival, a change in an intracellular signaling pathway, antigen expression, gene upregulation, gene downregulation, or a phenotypic change in response to an agent.

In a preferred embodiment of the invention, the cell, cell population, tissue, or whole organism is diseased or is derived from a diseased source. The diseased cell population, tissue, or whole organism can be diseased with any disease, malady, infection, and the like. Preferably, the disease is cancer, diabetes, an autoimmune disease, osteoporosis, or lung disease. Alternatively, the diseased cell population, tissue, or whole organism is infected with a parasite, virus, or bacteria. The diseased cell population, tissue, or whole organism can also be wounded, burned, scarred, or in a state of healing. The diseased cell also can be a protozoan, a nematode, *T. cruzi,* and leishmania.

The precise techniques for detecting the assayed activity will depend on the activity itself. For instance, if the activity is cell growth, then detection of the activity may simply comprise a cell count using a hemocytometer, visual inspection, or radioactive isotope uptake. If the activity is the production of a color-tagged product, then detection may involve detection of the color via ultraviolet-visible (UV-VIS) spectroscopy. It is well within the ordinary skill for one to determine suitable techniques for detecting the assayed activity.

Once the activity is detected, at least one entity-ligand-support complex having the entity, which exhibited the detected activity, is selected. The term "selecting" and words stemming therefrom as used herein refers to the identification of the entity-ligand-support complex, or the pool within which it resides. In the latter case, the assay may be repeated with just the constituents of the selected pool until a single entity-ligand-support complex is selected.

An advantage of the inventive methods is the ability to identify and/or characterize active entities on the basis of biological, physical, biochemical, or chemical activity, without prior knowledge of the entity's molecular identity. Accordingly, the entity can display a biological activity and need not undergo processing (e.g., heat-inactivation) prior to practicing the inventive methods. Also, there is no need to remove more abundant proteins like albumin or active entities like immunoglobulins. Likewise, the ability of an entity to affect more specific cellular functions (e.g., production of particular proteins or other cellular constituents) might be enhanced or diminished in the assay medium, thereby providing valuable characteristics of the entity. Furthermore, the entity might be a cell that proves resistant to cytotoxic agents. Thus, the invention provides methods for the identification of novel active entities or unknown active entities (i.e., proteins not identified prior to practicing the inventive method) with specific biological activities. Also, the invention provides identification of novel or unknown biological activities for known proteins.

The way in which the methods can achieve identification of the active entities can involve determining the chemical identity of the ligand(s) that bind(s) to the entity exhibiting the assayed activity. Suitable methods of determining the chemical identity of the ligand(s) are known in the art and include, for example, mass spectrometry, Edman degradation sequencing, and the like. Once ligands have been identified as having specificity for particular entities, those ligands can be resynthesized and used to capture, isolate, detect, and/or characterize entities using, for example, chromatographic separation. To this end, the inventive methods further comprise providing multiple copies of the identified ligand and attaching each copy of the identified ligand to a support, thereby obtaining multiple ligand-support complexes. The multiple-ligand support complexes are allowed to contact a composition containing multiple copies of the entity under conditions that allow the ligand-support complexes to bind to multiple copies of the entity, thereby forming multiple entity-ligand-support complexes. The entities are dissociated from the entity-ligand-support complexes and, if desired, subjected to additional rounds of screening and/or characterization. The composition containing multiple copies of the entity used to isolate and purify the entity can be the same as the mixture that was originally used to identify the entity as an active entity. For instance, if plasma was the mixture that was originally contacted with the ligand-support complexes, then the same source of plasma can be used to isolate and purify the entities in subsequent steps.

After the active entities have been isolated and purified, the method can further comprise the step of determining the chemical or physical identity of the entity or at least further characterizing the entity, such as by performing mass spectrometry of the entity. The term "characterization" and words related thereto as used herein refer to the identification of any distinctive quality or trait of a entity, and do not require that the precise chemical identity, e.g., the molecular formula, chemical structure, conformation, nucleotide sequence or amino acid sequence, of the entity is elucidated.

Additionally, the identified ligands also can be used in diagnostic assays, to immobilize or selectively transfer entities, and as pseudo- or synthetic receptors (see, e.g., Still, Acc. Chem. Res., 29: 155-163 (1996)). Additionally, the ligands themselves can be used as therapeutic agents, catalysts, and the like.

### EXAMPLES

The following examples further illustrate the invention but, of course, should not be construed as in any way limiting its scope.

For convenience, the following abbreviations are used in the examples herein: DEPFMU, 6,8-difluoro-4-methylumbelliferyl; ICAT, isotope coded affinity tag; UV-VIS, ultraviolet-visible; SAP, streptavidin-alkaline phosphatase; FITC, fluorescein isothiocyanate; ATCC, American Type Culture Collection; IL-2, interleukin-2; PNPP, p-nitro phenyl phosphate; PPV, porcine parvovirus; PVDF, polyvinylidene fluoride; AP, alkaline phosphatase; and PI, propidium iodide.

Example 1

This example demonstrates the use of the inventive methods to screen a mixture for cytotoxic factors.

The ability of a cytotoxic factor to induce cell death after being fractionated onto beads was determined. In this assay, beads to which anti-mouse TNF-α antibodies (Pharmingen) were bound, were incubated with purified mouse TNF-α (Pharmingen, San Diego, CA). Control beads were not incubated with TNF-α. In addition, controls included adding soluble TNF-α to the medium and incubating beads without antibody with TNF-α to ensure that there was no carryover of unbound TNF-α due to poor washing. The effect of plain beads was also measured. The indicated number of cells were plated per well in 24 well plates, and approximately 100 beads were added to each well of WEHI 164 cells (ATCC). After incubation with the beads for 48 hours, the indicated % cell death was observed by hemocytometer counting and trypan blue exclusion to indicate the number of dead cells (Table 1).

**Table 1. Cytotoxicity Assay of TNF-α beads**

| | Starting Cell No. | % Dead |
|---|---|---|
| Soluble TNF-α | 100,000 | 98 |
| Anti-TNFα beads alone | 100,000 | 0.5 |
| Sham incubation | 100,000 | 0.3 |
| Plain beads | 100,000 | 4.1 |
| | 50,000 | 0.1 |
| | 25,000 | 7.7 |
| TNF-α coupled beads | 100,000 | 9.1 |
| | 50,000 | 29.4 |
| | 25,000 | 12.5 |

This example demonstrated that sufficient amounts of protein eluted from these high-affinity ligands and caused detectable cell death in cultures of susceptible cells. These data support the thesis that active factors can be detected in multiple assays for biological function with defined endpoints.

Example 2

This example demonstrates the use of the inventive methods to screen cytokine-spiked plasma for factors that support cell growth.

The cells used in this assay were NK-92 cells (obtained from ATCC, Manassas, VA). The NK-92 cell line is a human cytotoxic T-cell line, which requires exogenous Interleukin-2 (IL-2) in order to grow. Normal cell culture medium containing serum or healthy human plasma does not have IL-2 to support NK-92 growth. Therefore, NK-92 cells normally are maintained in culture in medium supplemented with 5 ng/ml recombinant mouse IL-2.

As a model for beads bearing an IL-2 specific ligand, 300 µg of rat anti-human IL-2 antibody (Pharmingen, San Diego, CA) was cross-linked onto 400 µl of Protein G sepharose beads (Pierce, Rockford, IL). The beads were incubated overnight with a mixture of plasma into which a natural, secreted cytokine mixture was spiked. This mixture contained several cytokines and was derived from human monocytes and leukocytes that had been induced to secrete cytokines with phytohemagglutanin and ciprofloxacin. Media from these induced cells was subsequently pooled. The concentration of IL-2 in the spiked plasma was approximately 4 ng/ml. The beads were washed with ∼50 column volumes of PBS + 0.1% Tween-20 (Sigma Aldrich, St. Louis, MO). Beads that were incubated with 4 ng/ml pure recombinant human IL-2 were also included. Controls were beads bearing the antibody that were not incubated with IL-2 as a negative control and cultures to which soluble IL-2 was added as a positive control.

NK-92 cells were plated at 1x10⁵ cells per well in 24-well plates and allowed to grow in media without added IL-2 for 24 hours to deplete the intrinsic IL-2 stores. Approximately 100 beads were added to each well. The cultures were allowed to grow for 96 hours. Cells were collected from each culture and the cell number determined by counting live cells in a hemocytometer, excluding dead cells by trypan blue exclusion. Wells in which IL-2 was provided to the cells from ligands, either as recombinant protein or purified from the secreted mixture, showed an 8- to 12-fold increase in cell number compared with the negative control. Thus, these data demonstrate that a protein was fractionated from a highly complex mixture on a high-affinity ligand, the protein diffused from the ligand at a sufficient rate and concentration into media, and its presence was detected by its ability to support the growth of dependent cells.

These results indicate that sufficient protein diffused from a high affinity ligand and its activity was detected in a relevant cell-based assay. These results can be extrapolated to predict that an unknown protein that supports growth could be identified through its activity on cells in culture and the ligand that binds that protein identified, produced in bulk, and the active protein purified by affinity chromatography, identified, and characterized.

Example 3

This example demonstrates the use of the inventive methods to identify growth factors.

Complex cytokine mixtures have been screened for proteins that support the survival of NK-92 cells using a library of hexamer peptide ligands. A natural, secreted cytokine mixture derived from isolated lymphocytes and monocytes (ImmunoRx, Inc, Farmington, NY) was used as a starting material. This mixture contained many cytokines that are released in response to biological induction and are not present in normal sera or culture media. The endpoints of this assay are both biological and fluorescent as described in Example 2. 11,000 beads from a hexamer library synthesized via a cysteine derivative on a backbone of ToyoPearl 605-M epoxy beads (synthesized by Peptides International, Louisville, KY) were incubated with the cytokine mixture. The unbound and weakly bound proteins were removed by washing with PBS (150 mM NaCl, pH 7.4). 20 - 50 beads were incubated with 40 µl of NK-92 cells that had been plated at 2.4x10⁴ cells/ml in a well of a 384-well microtiter plate. The plates were maintained at 37°C for 48 hrs. Approximately 30 clumps of growing cells were observed in 20 wells. In some cases a large clump of cells grew in close association with a bead. There were several beads in the immediate vicinity which had no cells growing near them, and significant patches of dead cells were indicated by propidium iodide (PI) uptake. One of these beads (and a few others from similar wells that supported growth) was collected and the presence of IL-2 on the beads was confirmed by modified antibody detection in a "bead blot" assay. Briefly, the beads are arrayed in agarose, the proteins transferred off the beads onto a PVDF membrane by capillary transfer in elution buffer, and the membrane probed with anti-IL-2 antibodies to detect IL-2. Eleven beads, including three potential positives were recovered, cleaned, re-incubated with the identical starting material and cultured individually with the same cell line. Two of three potential positive beads reconfirmed their activity in the deconvolution assay. An additional bead was sequenced and the associated ligand identified as the sequence GVASED (SEQ ID NO: 9). A resin with this ligand was synthesized and found to bind IL-2. The complete starting material will be fractionated on the resin and the bound proteins will be analyzed to identify additional proteins that may be purified on the resin and which contribute to the activity.

Several aspects of this type of functional screening experiment can be extrapolated to other screens for biological activity. Although IL-2 is known to be present in the starting material there are also numerous other cytokines present at various levels on the beads and in the assay that may be contributing to cell survival. NK-92 cells have been tested for their lack of responsiveness to IL-1 and several other known cytokines; however, they may be responsive to other, as yet undiscovered cytokines that are present in the starting material. Most importantly, screening for factors that are protective against cytotoxic agents and poisons can clearly be accomplished in this type of assay in which cell growth and PI exclusion are endpoints.

Example 4

This example demonstrates the use of the inventive methods to screen spiked plasma for alkaline phophatase enzyme activity.

Streptavidin-alkaline phosphatase (500 ng) (SAP-Sigma-Aldrich) was spiked into 1 ml of pooled human plasma. The spiked plasma was incubated with ToyoPearl AF-Amino-650M beads (Tosoh BioSciences, Montgomeryville, PA) either with or without the ligand HPQFLS (SEQ ID NO: 1) (synthesized at Peptides International, Louisville, KY), a sequence known to bind streptavidin. The beads were allowed to incubate with spiked or unspiked plasma for 1 hr at room temperature, after which they were washed with HEPES buffer with 0.1% Tween-20, pH 7.2. Ligand-bearing beads were also incubated with saline alone as a control. The beads were distributed into wells of a 384-well plate with 6 HPQFLS beads per well and 120 µl of HEPES buffer containing 250 mM NaCl and 0.05% Tween-20 buffer. The beads were allowed to incubate for 30 hours at room temperature.

The liquid was removed from the wells and tested for the presence of alkaline phosphatase activity with p-nitro phenyl phosphate (PNPP) (Sigma-Aldrich), a colored phosphatase substrate. Yellow color developed only in those wells with buffer from ligand beads that had been exposed to AP-spiked plasma; no color was observed in the control wells. To confirm that protein did, in fact, elute from the beads, a FITC-labeled streptavidin (Pierce) was also incubated with the HPQFLS (SEQ ID NO: 1) beads as described above. The beads were then incubated with 120 µl of 20 mM citrate 140 mM NaCl buffer for 21 hours. The buffer was collected, and fluorescence was measured in a fluorometer. The increase over baseline was 11.6%. After an additional 3 days of elution, the fluorescence increased 22.7% over baseline.

These results demonstrate that a protein with enzymatic activity was fractionated onto a high-affinity ligand and eluted from the ligand, and that the presence of the protein in solution was measurable measured based on its biochemical activity.

Example 5

This example demonstrates the use of the inventive methods to screen plasma for melanoma specific antibodies.

The cell line used in this assay was the human, malignant melanoma cell line SK-MEL 28. The combinatorial library was synthesized on ToyoPearl 650-M epoxy resin from Tosoh BioScience. This library was designed to have the ligands linked to the resin through the sulfhydryl group of a cysteine derivative. The starting material used as a source of IgG, IgA and IgM antibodies was a plasma fraction I+II+III paste that had been further fractionated to remove fibrinogen. The beads were incubated with paste and washed, and between 50 -150 beads were incubated with 40 µl SK-MEL28 cells that had been plated at 1x10⁴ cells/ml in wells of a 384-well plate. The media contained propidium iodide (PI), a red-fluorescent dye that is taken up only by dead cells. Images of each well were taken at 1 hour intervals for 160 hours with single cell resolution. The images were analyzed for uptake of PI over the time period, and wells in which a cumulative increase in PI uptake that was 2 standard deviations above the mean of the population were selected as being of particular interest. The beads in these wells were harvested, cleaned, re-loaded with a fresh aliquot of the original starting material, and incubated with 1-2 beads per well with the same concentration of cells and imaged in the same way as the original assay. Beads in wells in which an increase in death above 2 standard deviations above the mean of the population was seen were collected and the associated ligand sequenced.

An increase in death in a well with a bead or in the vicinity of a specific bead indicated that a cytotoxic antibody was bound to the ligand. Any positive wells were deconvoluted by diluting the beads in the well to a single bead per well and repeating the assay. This confirmed the result and indicated the specific bead that bound the protein, whose associated ligand was sequenced. If a single bead was identified in a well without deconvolution, that bead was collected and the associated ligand sequenced.

Example 6

This example demonstrates the use of the inventive methods to screen a mixture of antibodies for antibodies of an immunized mammal.

Human plasma-derived intravenous immunoglobulin (IgG) was manufactured from pools of up to 60,000 donors and included vast varieties of antibodies with diverse affinities, some of which bind to cell surface receptors. Populations of antibodies can also be raised that are directed against cell surface receptors by immunizing mice with membrane preparations. The antibodies can be used as screening starting materials to identify their receptor epitopes in methods such as the "bead blot" receptor-binding antibodies may be useful for the upregulation of PON1. Specific epitopes for antibodies in a differential screen were identified using purified IgG preparations from normal and immunized mice. One cohort of mice was immunized with ovalbumin; unimmunized mice were used as controls. IgG preparations from each group were purified from pooled sera using affinity chromatography on protein G sepharose (Pierce), and the two populations were differentially labeled with either Alexa 488 (green) or Alexa 568 (red) dye (Molecular Probes, Eugene OR). The two IgG populations were then mixed and incubated with a library of combinatorial hexamer ligands. The beads were washed extensively and observed under a fluorescence microscope. The majority of beads that fluoresced had both red and green signals, indicating that they bound antibodies that were present in both populations. Beads that fluoresced only red were indicative of antibodies that were present only in the immunized population. These were collected and sequenced. Several showed very strong sequence similarity to ovalbumin, e.g. **ILRVIR** (SEQ ID NO: 2) has homology with the ovalbumin sequence RTINKVVRF (SEQ ID NO: 3), **IFDKVQG** (SEQ ID NO: 4) homology with **RFDKLPG**FG (SEQ ID NO: 5) and **PPFRIH**G (SEQ ID NO: 6) homology with **MPFRVITE** (SEQ ID NO: 7). Several of the remaining decoded sequences had high homology with bacterial sequences that are believed to have arisen from the Freund's adjuvant used during immunization.

Example 7

This example demonstrates the use of the inventive methods to identify enzymes bound to libraries by measuring paraoxonase activity.

A ligand that binds HDL was screened for its ability to purify paraoxonase from plasma by measuring the enzyme's activity. Paraoxonase is implicated in protecting against the build up of atherosclerotic plaque, as well as detoxification of some nerve poisons (sarin) and insecticides. 5 µg of the ligand 2'-naphthyl-alanineWLHAN (SEQ ID NO: 8) was incubated with 100 µl of 1:10 diluted rabbit serum in PBS for one hour at 37° C. The beads were centrifuged to remove the supernatant and the supernatant stored in eppendorf tubes. The resin bead pellets were resuspended in an equal volume of paraoxonase assay buffer. Paraoxonase activity of the supernatants was measured using a sensitive, specific, fluorescent substrate. 100 µM DEPFMU (6, 8-difluoro-4-methylumbelliferyl) was mixed with 10 µl supernatant, 10 µl of bead suspension, or 10 µl serum in a standard microtiter plate for 20 minutes at 37° C. Hydrolysis of DEPFMU was quantified by measuring fluoresce at 355 nm emission and 460 nm excitation using a commercial fluorometer. Hydrolysis was quantified compared with a standard curve of DEPFMU activity. 30% of the starting activity was detected in wells with the resins, whereas less than 4 % of the original paraoxonase activity remained in the serum. These data indicate that ligands that bind an enzyme can be detected through that enzyme's activity associated with a bead, and that enzymes themselves can be discovered from complex mixtures using this assay to screen for activity.

Example 8

This example demonstrates the use of the inventive methods to screen libraries for ligands that bind virus based on screening for virus-associated cytotoxicity.

Ligands that bind porcine parvovirus (PPV) were identified using the inventive methods. This assay was a modification of the classical plaque assay, in which infectivity is measured by viral plaques formed by lysis of susceptible mammalian cells in culture. As a model for ligands that bind virus, 2 µl of polyclonal porcine anti-PPV antibody was conjugated to Protein A sepharose beads according to the manufacturer's protocol (Pierce). The beads were incubated with 8 logs of PPV purified from PK13 cells by serial centrifugation. These beads were mixed with low-melting point agarose and spread over the surface of a 70% confluent culture of PK 13 cells. Following five days incubation at 37°C/ 5% CO₂, the live cells were stained with Neutral Red stain. There were evident areas of cell death surrounding beads that had bound virus. These results demonstrate that the controlled diffusion of active entities (viruses) from beads indicated the active bead.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising, " "having, " "including, " and "containing" are to be construed as open-ended terms (i.e., meaning "including, but not limited to, ") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A method of screening a mixture for active entities, which method comprises:
(i) providing a plurality of different ligands, wherein each ligand is attached to a support to form a plurality of ligand-support complexes,
(ii) contacting the ligand-support complexes with a mixture comprising a plurality of entities under conditions that allow at least one entity to bind to at least one ligand-support complex, thereby forming more than one entity-ligand-support complex,
(iii) separating more than one entity-ligand-support complex from the unbound entities;
(iv) assaying the activity of the entity, wherein the entity may be dissociated partially or completely from an entity-ligand-support complex separated in step (iii), and wherein the activity assayed is not solely binding of the entity to the ligand-support complex;
(v) detecting the activity, and
(vi) selecting at least one entity-ligand-support-complex that bound the entity that exhibited the detected activity,
whereupon a mixture is screened for active entities,
wherein the step of separating more than one entity-ligand support complex from the unbound entities does not comprise transferring the entity from a first matrix to a second matrix.

2. The method of claim 1, wherein the ligands are selected from the group consisting of proteins, peptides, amino acids, nucleic acids, carbohydrates, lipids, drugs, synthetic inorganic compounds, synthetic organic compounds, isoforms of any of the foregoing, cells, bacteria, viruses, yeast, and combinations of any of the foregoing.

3. The method of claim 2, wherein the ligands are peptides, and the peptides are generated by combinatorial approaches.

4. The method of claim 1, wherein the support comprises a material selected from the group consisting of polymethacrylates, polyacrylates, agarose, polyacrylamides, dextran, cellulose, polysaccharides, nitrocellulose, silicon, styrene, polyethylene-coated polystyrene, metal, polyvinyldifluoride, nylon, and combinations of any of the foregoing.

5. The method of claim 1, wherein the mixture is a biological fluid, an environmental extract, or a composition comprising chemical compounds.

6. The method of claim 5, wherein the biological fluid is selected from the group consisting of blood, plasma, pooled plasma, intermediates from plasma fractionation, serum, a cell homogenate, a tissue homogenate, a conditioned medium, a fermentation broth, cerebrospinal fluid, urine, saliva, milk, ductal fluid, tears, perspiration, lymph, semen, umbilical cord fluid, and amniotic fluid.

7. The method of claim 5, wherein the biological fluid is a plasma-derived fraction comprising antibodies and anti-idiotype antibodies.

8. The method of claim 5, wherein the biological fluid is obtained from a host afflicted with a disease.

9. The method of claim 5, wherein the environment extract is selected from the group consisting of a soil extract, an extract from a naturally-occurring body of water, a sample of ice, air, ash, rock, or permafrost, and a swab from a building.

10. The method of claim 5, wherein the composition comprising chemical compounds comprises natural or synthetic chemical compounds.

11. The method of claim 1, wherein the entities are selected from the group consisting of proteins, peptides, drugs, antibodies, cells, synthetic molecules, organic compounds, protein complexes, bacteria, viruses, and fungi.

12. The method of claim 1, wherein the activity is a biological, physical, chemical, biochemical activity or any combination thereof.

13. The method of claim 12, wherein the activity is an enzyme activity or an inhibition of an enzyme activity.

14. The method of claim 12, wherein the activity is an effect on a cell, a cell population, a tissue, or a whole organism.

15. The method of claim 14, wherein the activity is an effect on a cell or a cell population and the effect is selected from the group consisting of cell migration, cell proliferation, cell death, cell differentiation, cell cycle entry, cell cycle arrest, apoptosis, cell lysis, growth arrest, cell survival, a change in an intracellular signaling pathway, antigen expression, gene upregulation, gene downregulation, and a phenotypic change in response to an agent.

16. The method of claim 14, wherein the cell, cell population, tissue, or whole organism is diseased.

17. The method of claim 16, wherein the diseased cell, cell population, tissue, or whole organism is diseased with cancer, diabetes, an autoimmune disease, osteoporosis, or lung disease, infected with a parasite, virus, or bacteria, wounded, burned, scared, or in a state of healing.

18. The method of claim 1, which further comprises, after step (iii) and before step (iv), a sub-pooling step, wherein at least one separated entity-ligand-support complex and the ligand-support complexes are separated into several pools.

19. The method of claim 1, which further comprises (vii) determining the chemical identity of at least one ligand to which at least one entity exhibiting the detected activity binds.

20. The method of claim 19, which further comprises
(viii) providing multiple copies of at least one ligand identified in step (vii),
(ix) attaching each copy to a support, thereby obtaining multiple ligand-support complexes,
(x) contacting the ligand-support complexes with a composition comprising multiple copies of at least one entity exhibiting the detected activity under conditions that allow the ligand-support complexes to bind multiple copies of at least one entity exhibiting the detected activity, thereby forming multiple entity-ligand-support complexes, and
(xi) dissociating the entities from the entity-ligand-support complexes, thereby recovering the entities from the composition.

21. The method of claim 20, wherein the composition of step (x) is the same as the mixture of step (ii).

22. The method of claim 20, which further comprises
(xii) determining the chemical or physical identity of the entity exhibiting the detected activity.

23. The method of claim 18, which further comprises, after the sub-pooling step and before step (iv), an eluting step, wherein the entities of the multiple entity-ligand-support complexes are dissociated from the complexes, and the ligand-support complexes are subsequently removed from the pools.

24. The method of claim 18, which method comprises, after the sub-pooling step and before step (iv), a step, wherein a semi-solid or viscous material is added to each pool, wherein the entity of at least one entitity-ligand-support complexes dissociates from the complex and diffuses into the material, thereby forming a concentration gradient of the entity, wherein the concentration of the entity gradually decreases as the distance from the ligand-support complex from which the entity dissociated increases.

25. The method of claim 1, further comprising characterizing the active entity and its binding ligand.

## Patentansprüche

1. Verfahren zum Screenen eines Gemischs im Hinblick auf aktive Entitäten, wobei das Verfahren Folgendes beinhaltet:
(i) Bereitstellen mehrerer verschiedener Liganden, wobei jeder Ligand an einem Träger angebracht ist, um mehrere Ligand-Träger-Komplexe zu bilden,
(ii) Inkontaktbringen der Ligand-Träger-Komplexe mit einem Gemisch, das mehrere Entitäten umfasst, unter solchen Bedingungen, dass sich wenigstens eine Entität an wenigstens einen Ligand-Träger-Komplex binden kann, so dass mehr als ein Entität-Ligand-Träger-Komplex gebildet wird;
iii) Trennen von mehr als einem Entität-Ligand-Träger-Komplex von den ungebundenen Entitäten;
(iv) Untersuchen der Aktivität der Entität, wobei die Entität teilweise oder vollständig von einem in Schritt (iii) getrennten Entität-Ligand-Träger-Komplex dissoziiert werden kann und wobei die untersuchte Aktivität nicht nur die Bindung der Entität an den Ligand-Träger-Komplex ist;
(v) Detektieren der Aktivität und
(vi) Selektieren von wenigstens einem Entität-Ligand-Träger-Komplex, der die Entität gebunden hat, die die detektierte Aktivität aufwies,
woraufhin ein Gemisch im Hinblick auf aktive Entitäten gescreent wird,
wobei der Schritt des Trennens von mehr als einem Entität-Ligand-Träger-Komplex von den ungebundenen Entitäten nicht das Übertragen der Entität von einer ersten Matrix zu einer zweiten Matrix beinhaltet.

2. Verfahren nach Anspruch 1, wobei die Liganden ausgewählt sind aus der Gruppe bestehend aus Proteinen, Peptiden, Aminosäuren, Nucleinsäuren, Kohlenhydraten, Lipiden, Arzneimitteln, synthetischen anorganischen Verbindungen, synthetischen organischen Verbindungen, Isoformen von beliebigen der Vorerwähnten, Zellen, Bakterien, Viren, Hefe und Kombinationen beliebiger der Vorerwähnten.

3. Verfahren nach Anspruch 2, wobei die Liganden Peptide sind und die Peptide durch kombinatorische Ansätze erzeugt werden.

4. Verfahren nach Anspruch 1, wobei der Träger ein Material umfasst, das ausgewählt ist aus der Gruppe bestehend aus Polymethacrylaten, Polyacrylaten, Agarose, Polyacrylamiden, Dextran, Cellulose, Polysacchariden, Nitrocellulose, Silizium, Styrol, Polyethylen-beschichtetem Polystyrol, Metall, Polyvinyldifluorid, Nylon und Kombinationen beliebiger der Vorerwähnten.

5. Verfahren nach Anspruch 1, wobei das Gemisch ein biologisches Fluid, ein Umweltextrakt oder eine Zusammensetzung ist, die chemische Verbindungen umfasst.

6. Verfahren nach Anspruch 5, wobei das biologische Fluid ausgewählt ist aus der Gruppe bestehend aus Blut, Plasma, Poolplasma, Intermediaten aus der Plasmafraktionierung, Serum, einem Zellhomogenat, einem Gewebehomogenat, einem konditionierten Medium, Fermentationsbrühe, Zerebrospinalflüssigkeit, Urin, Speichel, Milch, Drüsengangflüssigkeit, Tränenflüssigkeit, Schweiß, Lymphflüssigkeit, Samenflüssigkeit, Nabelschnurflüssigkeit und Fruchtwasser.

7. Verfahren nach Anspruch 5, wobei das biologische Fluid eine von Plasma abstammende Fraktion ist, die Antikörper und Anti-Idiotyp-Antikörper umfasst.

8. Verfahren nach Anspruch 5, wobei das biologische Fluid von einem an einer Krankheit leidenden Wirt gewonnen wird.

9. Verfahren nach Anspruch 5, wobei der Umweltextrakt ausgewählt wird aus der Gruppe bestehend aus einem Bodenextrakt, einem Extrakt von einem natürlichen Gewässer, einer Probe von Eis, Luft, Asche, Gestein oder Permafrost und einem Abstrich von einem Gebäude.

10. Verfahren nach Anspruch 5, wobei die Zusammensetzung, die chemische Verbindungen umfasst, natürliche oder synthetische chemische Verbindungen umfasst.

11. Verfahren nach Anspruch 1, wobei die Entitäten ausgewählt sind aus der Gruppe bestehend aus Proteinen, Peptiden, Arzneimitteln, Antikörpern, Zellen, synthetischen Molekülen, organischen Verbindungen, Proteinkomplexen, Bakterien, Viren und Pilzen.

12. Verfahren nach Anspruch 1, wobei die Aktivität eine biologische, physikalische, chemische, biochemische Aktivität oder eine beliebige Kombination davon ist.

13. Verfahren nach Anspruch 12, wobei die Aktivität eine Enzymaktivität oder die Inhibition einer Enzymaktivität ist.

14. Verfahren nach Anspruch 12, wobei die Aktivität eine Wirkung auf eine Zelle, eine Zellpopulation, ein Gewebe oder einen ganzen Organismus ist.

15. Verfahren nach Anspruch 14, wobei die Aktivität eine Wirkung auf eine Zelle oder eine Zellpopulation ist und die Wirkung ausgewählt ist aus der Gruppe bestehend aus Zellmigration, Zellproliferation, Zelltod, Zelldifferenzierung, Zellzykluseintritt, Zellzyklushemmung, Apoptose, Zelllyse, Wachstumshemmung, Zellüberleben, einer Änderung in einem intrazellulären Signalisierungspfad, Antigenexpression, Gen-Up-Regulation, Gen-Down-Regulation und einer phänotypischen Änderung als Reaktion auf ein Agens.

16. Verfahren nach Anspruch 14, wobei die Zelle, Zellpopulation, das Gewebe oder der ganze Organismus erkrankt ist.

17. Verfahren nach Anspruch 16, wobei die/das/der erkrankte Zelle, Zellpopulation, Gewebe oder ganze Organismus an Krebs, Diabetes, einer Autoimmunkrankheit, Osteoporose oder einer Lungenkrankheit erkrankt ist, durch einen Parasiten, ein Virus oder ein Bakterium infiziert ist, verwundet, verbrannt, vernarbt ist oder sich in einem Heilungszustand befindet.

18. Verfahren nach Anspruch 1, das nach Schritt (iii) und vor Schritt (iv) ferner einen Sub-Pooling-Schritt beinhaltet, wobei wenigstens ein getrennter Entität-Ligand-Träger-Komplex und die Ligand-Träger-Komplexe in mehrere Pools aufgeteilt werden.

19. Verfahren nach Anspruch 1, das ferner (vii) das Bestimmen der chemischen Identität von wenigstens einem Ligand umfasst, an den sich wenigstens eine Entität bindet, die die detektierte Aktivität aufweist.

20. Verfahren nach Anspruch 19, das ferner die folgenden Schritte beinhaltet:
(viii) Bereitstellen mehrerer Kopien von wenigstens einem in Schritt (vii) identifizierten Ligand,
(ix) Anbringen von jeder Kopie an einen Träger, so dass mehrere Ligand-Träger-Komplexe erhalten werden,
(x) Inkontaktbringen der Ligand-Träger-Komplexe mit einer Zusammensetzung, die mehrere Kopien von wenigstens einer Entität umfasst, die die detektierte Aktivität aufweist, unter solchen Bedingungen, dass die Ligand-Träger-Komplexe mehrere Kopien von wenigstens einer Entität binden können, die die detektierte Aktivität aufweist, so dass mehrere Entität-Ligand-Träger-Komplexe gebildet werden, und
(xi) Dissoziieren der Entitäten von den Entität-Ligand-Träger-Komplexen, wodurch die Entitäten von der Zusammensetzung wiedergewonnen werden.

21. Verfahren nach Anspruch 20, wobei die Zusammensetzung aus Schritt (x) dem Gemisch aus Schritt (ii) entspricht.

22. Verfahren nach Anspruch 20, das ferner (xii) das Bestimmen der chemischen oder physikalischen Identität der Entität beinhaltet, die die detektierte Aktivität aufweist.

23. Verfahren nach Anspruch 18, das ferner nach dem Sub-Pooling-Schritt und vor Schritt (iv) einen Elutionsschritt beinhaltet, wobei die Entitäten der mehreren Entität-Ligand-Träger-Komplexe von den Komplexen dissoziiert und die Ligand-Träger-Komplexe anschließend aus den Pools entfernt werden.

24. Verfahren nach Anspruch 18, wobei das Verfahren nach dem Sub-Pooling-Schritt und vor Schritt (iv) einen Schritt beinhaltet, bei dem ein halbfestes oder viskoses Material zu jedem Pool gegeben wird, wobei sich die Entität von wenigstens einem Entität-Ligand-Träger-Komplex von dem Komplex dissoziiert und in das Material diffundiert, so dass ein Konzentrationsgradient der Entität entsteht, wobei die Konzentration der Entität mit der Zunahme des Abstands vom Ligand-Träger-Komplex, von dem sich die Entität dissoziierte, allmählich abnimmt.

25. Verfahren nach Anspruch 1, das ferner das Charakterisieren der aktiven Entität und seines Bindungsligands beinhaltet.

## Revendications

1. Procédé de criblage d'un mélange pour des entités actives, lequel procédé comprend:
(i) fournir une pluralité de ligands différents, où chaque ligand est attaché à un support pour former une pluralité de complexes ligand-support,
(ii) mettre les complexes ligand-support en contact avec un mélange comprenant une pluralité d'entités dans des conditions qui permettent à au moins une entité de se lier à au moins un complexe ligand-support formant ainsi plus d'un complexe entité-ligand-support,
(iii) séparer plus d'un complexe entité-ligand-support des entités non liées,
(iv) analyser l'activité de l'entité, où l'entité peut être partiellement ou complètement dissociée d'un complexe entité-ligand-support séparé à l'étape (iii) et où l'activité analysée n'est pas seulement la liaison de l'entité au complexe ligand-support,
(v) détecter l'activité, et
(vi) sélectionner au moins un complexe entité-ligand-support qui se lie à l'entité qui a présenté l'activité détectée,
sur quoi un mélange est criblé pour des entités actives,
où l'étape consistant à séparer plus d'un complexe entité-ligand-support des entités non liées, ne comprend pas transférer l'entité d'une première matrice à une deuxième matrice.

2. Le procédé de la revendication 1, dans lequel les ligands sont sélectionnés parmi le groupe consistant en protéines, peptides, acides aminés, acides nucléiques, hydrates de carbone, lipides, médicaments, composé synthétiques inorganiques, composés synthétiques organiques, isoformes de l'un quelconque des éléments précédents, cellules, bactéries, virus, levure et des combinaisons de l'un quelconque des éléments précédents.

3. Le procédé de la revendication 2, dans lequel les ligands sont des peptides et les peptides sont produits par des démarches combinatoires.

4. Le procédé de la revendication 1, dans lequel le support comprend une matière sélectionnée parmi le groupe consistant en polyméthacrylates, polyacrylates, agarose, polyacrylamides, dextrane, cellulose, polysaccharides, nitrocellulose, silicium, styrène, polystyrène à revêtement polyéthylène, métal, difluorure de polyvinyle et des combinaisons de l'un quelconque des éléments précédents.

5. Le procédé de la revendication 1, dans lequel le mélange est un liquide biologique, un extrait de l'environnement ou une composition comprenant des composés chimiques.

6. Le procédé de la revendication 5, dans lequel le liquide biologique est sélectionné parmi le groupe consistant en sang, plasma, mélange de plasmas, intermédiaires provenant d'un fractionnement de plasma, sérum, un homogénéisat cellulaire, un homogénéisat tissulaire, un milieu conditionné, un bouillon de fermentation, liquide céphalo-rachidien, urine, salive, lait, liquide canalaire, larmes, sueur, lymphe, sperme, liquide du cordon ombilical et liquide amniotique.

7. Le procédé de la revendication 5, dans lequel le liquide biologique est une fraction dérivée de plasma comprenant des anticorps et des anticorps anti-idiotypes.

8. Le procédé de la revendication 5, dans lequel le liquide biologique est obtenu d'un hôte affligé d'une maladie.

9. Le procédé de la revendication 5, dans lequel l'extrait de l'environnement est sélectionné parmi le groupe consistant en un extrait de sol, un extrait d'un corps d'eau survenant naturellement, un échantillon de glace, d'air, de cendre, de roche ou de permafrost et un prélèvement d'un bâtiment.

10. Le procédé de la revendication 5, dans lequel la composition comprenant des composés chimiques comprend des composés chimiques naturels ou synthétiques.

11. Le procédé de la revendication 1, dans lequel les entités sont sélectionnées parmi le groupe consistant en protéines, peptides, médicaments, anticorps, cellules, molécules synthétiques, composés organiques, complexes protéiques, bactéries, virus et champignons.

12. Le procédé de la revendication 1, dans lequel l'activité est une activité biologique, physique, chimique, biochimique ou une combinaison de celles-ci.

13. Le procédé de la revendication 12, dans lequel l'activité est une activité enzymatique ou une inhibition d'une activité enzymatique.

14. Le procédé de la revendication 12, dans lequel l'activité est un effet sur une cellule, une population de cellules, un tissu ou un organisme entier.

15. Le procédé de la revendication 14, dans lequel l'activité est un effet sur une cellule
ou une population de cellules et l'effet est sélectionné parmi le groupe consistant en migration cellulaire, prolifération cellulaire, mort cellulaire, différenciation cellulaire, entrée dans le cycle cellulaire, arrêt du cycle cellulaire, apoptose, lyse cellulaire, arrêt de croissance, survie cellulaire, un changement dans un processus de signalisation intracellulaire, expression d'un antigène, régulation génique positive, régulation génique négative et un changement phénotypique en réponse à un agent.

16. Le procédé de la revendication 14, dans lequel la cellule, la population cellulaire, le tissu ou l'organisme entier est malade.

17. Le procédé de la revendication 16, dans lequel la cellule, la population cellulaire, le tissu ou l'organisme entier malade est atteint de cancer, de diabète, d'une maladie autoimmune, d'ostéoporose ou de maladie pulmonaire, est infecté par un parasite, un virus ou une bactérie, est blessé, brûlé, marqué de cicatrices ou dans un état de guérison.

18. Le procédé de la revendication 1, lequel comprend en outre, après l'étape (iii) et avant l'étape (iv), une étape de sous-regroupement à laquelle au moins un complexe entité-ligand-support séparé et les complexes ligand-support sont séparés en plusieurs regroupements.

19. Le procédé de la revendication 1, lequel comprend en outre (vii) déterminer l'identité chimique d'au moins un ligand auquel se lie au moins une entité présentant l'activité détectée.

20. Le procédé de la revendication 19, lequel comprend en outre:
(viii) fournir des copies multiples d'au moins un ligand identifié à l'étape (vii),
(ix) attacher chaque copie à un support, obtenant ainsi des complexes ligand-support multiples,
(x) mettre les complexes ligand-support en contact avec une composition comprenant des copies multiples d'au moins une entité présentant l'activité détectée dans des conditions qui permettent aux complexes ligand-support de se lier à des copies multiples d'au moins une entité présentant l'activité détectée, formant ainsi des complexes entité-ligand-support multiples, et
(xi) dissocier les entités des complexes entité-ligand-support, recueillant ainsi les entités de la composition.

21. Le procédé de la revendication 20, dans lequel la composition de l'étape (x) est la même que le mélange de l'étape (ii).

22. Le procédé de la revendication 20, lequel comprend en outre:
(xii) déterminer l'identité chimique ou physique de l'entité présentant l'activité détectée.

23. Le procédé de la revendication 18, lequel comprend en outre, après l'étape de sous-regroupement et avant l'étape (iv), une étape d'élution à laquelle les entités des complexes entité-ligand-support multiples sont dissociées des complexes et les complexes ligand-support sont subséquemment retirés des regroupements.

24. Le procédé de la revendication 18, lequel procédé comprend, après l'étape de sous-regroupement et avant l'étape (iv), une étape à laquelle une matière semi-solide ou visqueuse est ajoutée à chaque regroupement, où l'entité d'au moins un complexe entité-ligand-support se dissocie du complexe et se diffuse dans la matière formant ainsi un gradient de concentration de l'entité, où la concentration de l'entité diminue graduellement au fur et à mesure que la distance du complexe ligand-support duquel l'entité s'est dissociée, augmente.

25. Le procédé de la revendication 1, comprenant en outre caractériser l'entité active et son ligand de liaison.


### SEQUENCE LISTING

<110> The American National Red Cross et al.
<120> METHOD FOR IDENTIFYING INDIVIDUAL ACTIVE ENTITIES FROM COMPLEX MIXTURES
<130> 222362
<150> US 60/395,038
   <151> 2002-07-11
<160> 9
<170> PatentIn version 3.2
<210> 1
   <211> 6
   <212> PRT
   <213> Artificial
<220>
   <223> Synthetic
<400> 1
   His Pro Gln Phe Leu Ser
   1               5
<210> 2
   <211> 6
   <212> PRT
   <213> Artificial
<220>
   <223> Synthetic
<400> 2
   Ile Leu Arg Val Ile Arg
   1               5
<210> 3
   <211> 9
   <212> PRT
   <213> Artificial
<220>
   <223> Synthetic
<400> 3
   Arg Thr Ile Asn Lys Val Val Arg Phe
   1               5
<210> 4
   <211> 7
   <212> PRT
   <213> Artificial
<220>
   <223> Synthetic
<400> 4
   Ile Phe Asp Lys Val Gln Gly
   1               5
<210> 5
   <211> 9
   <212> PRT
   <213> Artificial
<220>
   <223> Synthetic
<400> 5
   Arg Phe Asp Lys Leu Pro Gly Phe Gly
   1 5
<210> 6
   <211> 7
   <212> PRT
   <213> Artificial
<220>
   <223> Synthetic
<400> 6
   Pro Pro Phe Arg Ile His Gly
   1 5
<210> 7
   <211> 8
   <212> PRT
   <213> Artificial
<220>
   <223> Synthetic
<400> 7
   Met Pro Phe Arg Val Ile Thr Glu
   1 5
<210> 8
   <211> 5
   <212> PRT
   <213> Artificial
<220>
   <223> Synthetic
<400> 8
   Trp Leu His Ala Asn
   1 5
<210> 9
   <211> 6
   <212> PRT
   <213> Artificial
<220>
   <223> Synthetic
<400> 9
   Gly Val Ala Ser Glu Asp
   1               5